# EUROPEAN PATENT APPLICATION

(11) **EP 1 118 851 A2**
(43) Date of publication of application: **25.07.2001**
(21) Application number: 01830009.5
(22) Date of filing: 11.01.2001
(51) Int. Cl.: G01N 3/14, G01N 3/08

(54) **A device for measuring the compression that can be exerted by a hosiery article**

(30) Priority: 21.01.2000 IT FI000008
(71) Applicant: Filodoro Calze S.p.A., 46040 Casalmoro (Montova) (IT)
(72) Inventor: Pirlitescu, Marius, 46041 Asola, Mantova (IT); Giudici, Davide, 46040 Casalmoro, Mantova (IT); Quaranta, Marco, 46043 Castiglione Delle Stiviere (IT); Bignotti, Rudi, 46043 Castiglione Delle Stiviere (IT)
(74) Representative: Bardini, Marco Luigi

(57) **Abstract**

A device for measuring the compression that can be exerted by a hosiery article, capable of displaying and recording the compression data both in a static and in a dynamic condition, uses a detection system with two-dimensional sensors (3), each comprising a matrix arrangement of load cells. Sensors (3) are connected to the outside of at least one axially symmetrical and radially extensible member (2, 102), on which the article is to be arranged. Member (2, 102) can consist of a number of rigid shells (1, 101), radially driven by independent motors controlled by programmable means, according to predetermined working steps. Besides, said means are capable of reading and processing the output signals of the sensors (3). In this way, motion cycles of member (2, 102) can be set and programmed in order to carry out different kinds of measurement.

## Description

The present invention relates to the field of hosiery, and more precisely it concerns a device for measuring the compression that can be exerted by a stocking, a pantyhose or the like.

The possibility of measuring precisely the radial compressing force, hereinafter referred to simply as compression, that a hosiery article, when worn, can exert on the wearer's legs is extremely important, both when designing the article (trial products being evaluated) and when manufacturing it, in order to check, statistically, whether the same complies with the design features which are sought and presented in the advertisements.

The system which is used at present for measuring the compression of a hosiery article makes use of a suitably adjusted load cell, which is applied over the outside of the fabric, kept extended. In fact, when the measuring operation has to be carried out, the stocking - or the like - is placed on a device consisting of a pair of bars which can depart each other in a compass-like arrangement. A leg portion of the article is inserted on the bars, having a set length, so as to be extended by them on a plane.

In further detail, whereas one of the bars is fixed, the other is pivotable and supports mock-ups emulating the shape of the calf and thigh of a leg. A third mock-up is connected to the articulation end of the arrangement, emulating the foot. Depending on the article size to be reproduced, the device is adjusted by replacing the mock-ups of the movable bar and changing the overall angular displacement between the bars. A carriage is slidable along he fixed bar and supports a load cell sensor for measuring the compression. The measurement is carried out by applying a sensor head on the fabric, in correspondence to the fixed bar. The numerical indication of the compression value is read on a display mounted on the carriage.

This compression measuring system is not satisfactory for a number of reasons. First of all, the measurement is not reliable due to the frictions generated by the plane extension procedure. Such frictions cause remarkable unsystematic errors, as confirmed by the fact that values collected by different operators on the same article do not correspond, and that the results of repeated measurements taken by the same operator fall within a comparatively wide range.

Although the device has mock-ups to be connected to the bars in order to reproduce the silhouette defined by each different size, the bidimensional extension does not actually succeed in simulating the real condition of the article when worn by the human leg (having a substantially elliptical symmetry). The defectiveness of the simulation unavoidably affects the reliability of the measurement.

Moreover, it has to be noticed that the numerical value detected by the sensor is assumed constant on the corresponding circumference of the leg. In other words, it is assumed that, for instance at the level of the calf, the compression is the same in the front area and in the back one. This modelling can not interpret the real circumstances correctly, generally speaking due to the asymmetry of the body figure, but also because new types of stockings, recently produced by the same applicant, accomplish a different compression along a predetermined circumference.

A further drawback of the known measurement system is given by the fact that the mock-up of the foot can not be replaced depending on the size considered and simulated. This results in a shifting of the stocking along the bars, and in the consequent altering further the measurement conditions with respect to the real ones.

Furthermore, the known system can not actually detect the compression of the elastic rim, either of a knee high or of a complete pantyhose. Analogously, it is not possible to ascertain the condition of the reinforced toe.

Finally, the traditional system can cope with static condition measurements only, not with dynamic ones. In fact, any attempt to achieve a dynamic simulation, by keeping the movable bar in motion, results in such a departure from a realistic condition that the reliability of the measurement is absolutely worthless.

All things considered, what the present device can provide is at the most, disregarding the systematic errors, measurements that can be used for comparing different articles. Conversely, it is absolutely unsuitable in order to ascertain the compression conditions that actually occur in each point of the article when worn.

The general object of the present invention is therefore to provide a device for measuring the compression that can be exerted by a hosiery article, capable of permitting precise and reliable measurements, indicating the actual compression that the article can exert when worn.

A particular object of the present invention is to provide a device of the above-mentioned kind, capable of measuring the compression substantially point by point.

A further particular object of the invention is to provide a device of the above-mentioned kind, permitting the carrying out of measurements in dynamic conditions.

Such objects are achieved with the device for measuring the compression that can be exerted by a hosiery article according to the present invention, having the essential features defined in the first of the appended claims.

The features and advantages of the device for measuring the compression that can be exerted by a hosiery article according to the present invention will be made clearer with the following description of an embodiment thereof, made purely by way of example and not limitative, with reference to the attached drawings in which:
- figures 1, 2, 3a and 3b schematically represent the measurement system on which the present invention is based;
- figure 4 is a schematic perspective view of a device according to a first embodiment of the invention;
- figure 5 shows the electric layout of the device of figure 1, by means of a flowchart diagram;
- figures 6a and 6b are schematic perspective views of a device according to a different embodiment of the invention, respectively when a minimum and a maximum size of the wearer are simulated;
- figures 7a and 7b are schematic perspective views from the above of one of the members forming the device of figures 6a and 6b, parts being omitted, respectively when a minimum and a maximum size of the wearer are simulated;
- figure 8 shows the member of figures 7a and 7b by way of a schematic perspective view from below;
- figures 9a and 9b are schematic perspective views of a foot member of the device of figures 6a and 6b, parts being omitted, respectively when a minimum and a maximum size of the wearer are simulated;
- figure 10 shows the electric layout of the device according to the embodiment of the invention of figures from 6a to 9b, by means of a flowchart diagram;

According to the invention, the compression exerted by a pantyhose or the like is measured by reproducing as realistically as possible, thanks to a radially extensible, axially symmetrical member, the contact relationship occurring between the elastic fabric and the leg when the pantyhose is actually worn. At the same time, the compression exerted by the pantyhose is assessed by way of a detection means the provision of which does not substantial affect the measure.

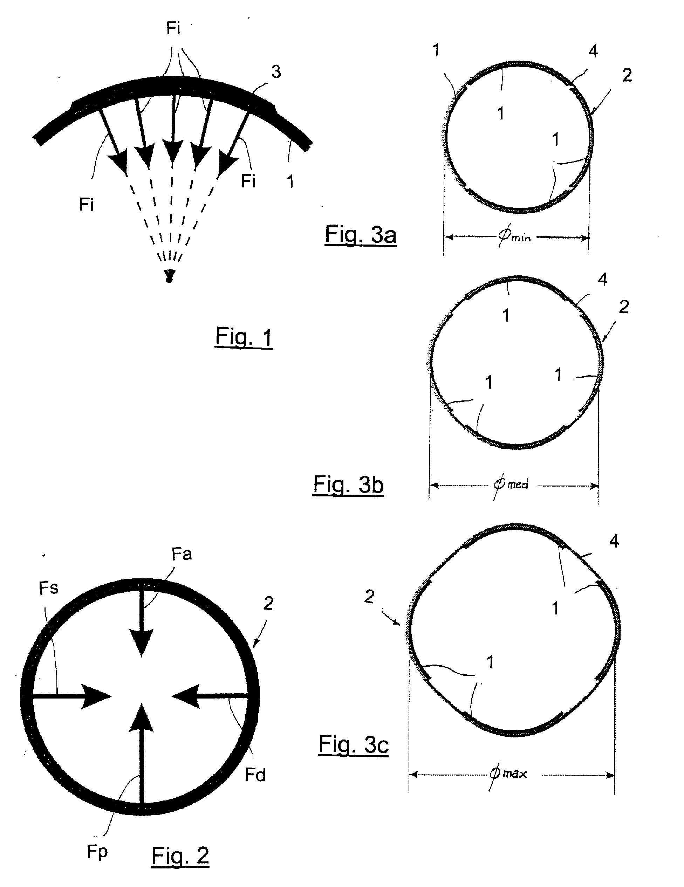

With reference to figure 1, the reproduction is carried out preferably by means of substantially shell-shaped elements 1 providing outer faces on which respective two-dimensional sensors 3 are placed. More precisely, sensor 3 comprises a matrix arrangement of micro load cells, each capable of measuring force Fi exerted thereon in a radial direction. This kind of sensors is *per se* known, and available on the market. For instance, the sensors manufactured by the US company TEKSCAN Inc of Boston (Mass) can be used.

The average among the forces Fi is assumed to be the average force exerted on shell 1. Thanks to such arrangement, if some cells give errors, the effect thereof is minimised by the output of the others. In other words, thanks to the great number of cells, the overall signal is remarkably increased and the noise, inherent to any measurement, is drastically reduced.

The average compression can be worked out, rather than on the whole shell 1, on respective spots thereof. If high surface resolution sensors 3 are used - that is to say sensors having a high micro-cell density - said spots can be extremely small, so as to make it possible to measure the compression substantially point by point.

Then, according to what is shown by figure 2, the invention provides for the combination of a number of shells 1, e. g. four shells defining each a 90° solid angle, in order to form a rigid drum 2 simulating a portion of a human leg. If a tubular portion of a pantyhose is arranged on drum 2, assuming that the minimum size of the drum is bigger than that of the unworn tubular portion, a compression action is generated and detected by sensors 3 according to the above described approach.

Still referring to figure 2, respective average compression forces exerted on shells 1 are indicated. Namely, average compression forces on the front, back, right and left shells are indicated at FA, FP, FD and FS, respectively, the position of the tubular portion when worn being referred to. Thus, it is possible to obtain the compression value in various points of the pantyhose circumference, without being compelled to assume that the compression is constant along the entire circumference, according to a hypothesis that is clearly incorrect.

The noise caused by each sensor 3, due to its own width, can be disregarded, since such width is very small (smaller than width of drum 2 by several orders of magnitude). In other words, the compression measured by sensors 3 can be assumed to be the same that is actually exerted on drum 2.

According to the invention, a device exploiting the above described measurement system can be delineated by the general scheme of figures from 3a to 3c. A tubular portion 4 is arranged on drum 2 formed by four shells 1, on the outer faces of which respective sensors, not shown, are applied. Shells 1 are controlled by a drive system, which can displace them in a radial direction. The displacement can be the same for all the shells, like in the depicted example, or different for each of them. Consequently, the width of drum 2 varies from a minimum value min (figure 3a) to a maximum value max (figure 3c), through intermediate values med (figure 3d). As a result, the variation in width of tubular portion 4 along the length of the leg, as well as according to the size, are reproduced.

In point of fact, the compression value detected by sensors 3 does not exactly correspond to that exerted by a leg portion of the corresponding width, because, especially when the width approaches max, drum 2 actually becomes similar to a prism with rounded edges. On the other hand, it can be easily understood that said error is certainly not a major one.

When reducing the invention to practice, according to a first embodiment, an automated device is provided, by means of which a single type of measurement is carried out in each session, e.g. the elastic band of a knee-high, or of a pantyhose, the tip of the tubular portion etc., each time using shells 1 having appropriate features, with respective sensors previously adjusted and balanced.

In this case, the influences on the compression deriving from the mutual interaction between the different parts of the tubular portion are disregarded. This because it is not the shape of the entire leg to be simulated, but only a spot surrounding the measured area. On the other hand in many circumstances, and namely when the compression of the rim is measured, the high detected value ensures for a fairly reasonable assessment even though the influence of the remaining parts of the article is disregarded.

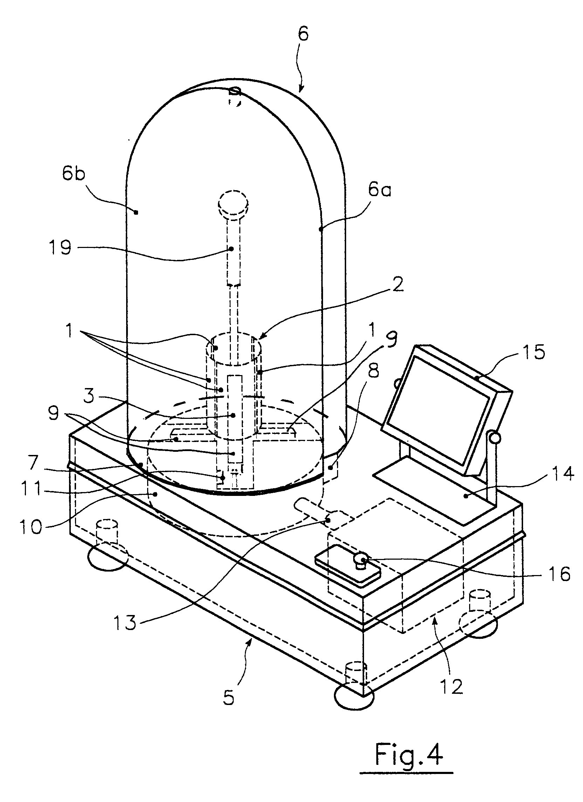

With reference to figure 4, said device comprises a basement 5, with protection and support functions, from which drum 2 stands, enclosed by a dome-shaped coaxial case 6, made of a transparent plastic or vitreous material. In further detail, case 6 consists of a fixed part 6a and a movable one 6b, which turns around the central axis of the case - moving along a circular guide 7 - in order to permit the access to drum 2. A proximity sensor 8 detects the closed or open condition of case 6.

A telescopic rod 19 also stands from basement 5, coaxially to drum 2 and case 6, for stretching pantyhose or a hosiery tubular portion arranged on drum 2. Shells 1, externally provided with sensors 3, are supported by respective radially sliding jaws 9 of a self-centering platform 10, of the type which is used for locking the workpiece in a machine-tool, supported by basement 5 as well. Three proximity sensors 11, only one of which is visible in the figure, firmly fixed on the top part of platform 10, establish the run-end stop of jaws 9, and thus the maximum width which can be accomplished by drum 2.

A control unit of the device is schematically represented by a box 12 and comprises a step motor which, via a drive shaft 13 extending from box 12, drives jaws 9 by means of a screw mechanism. the operation of the motor is controlled by a PLC synchronised with software for controlling sensors 3. A position transducer connected to the shaft of the step motor is entitled to the feedback function. The PLC is programmed via a user interface comprising a keyboard 14 and a display 15 for displaying the results of the measurements. Furthermore, said interface comprises two switches, not shown, for controlling an electric feed of the motor and for an emergency stop. A further emergency stop switch 16 is directly located on the outside of basement 5.

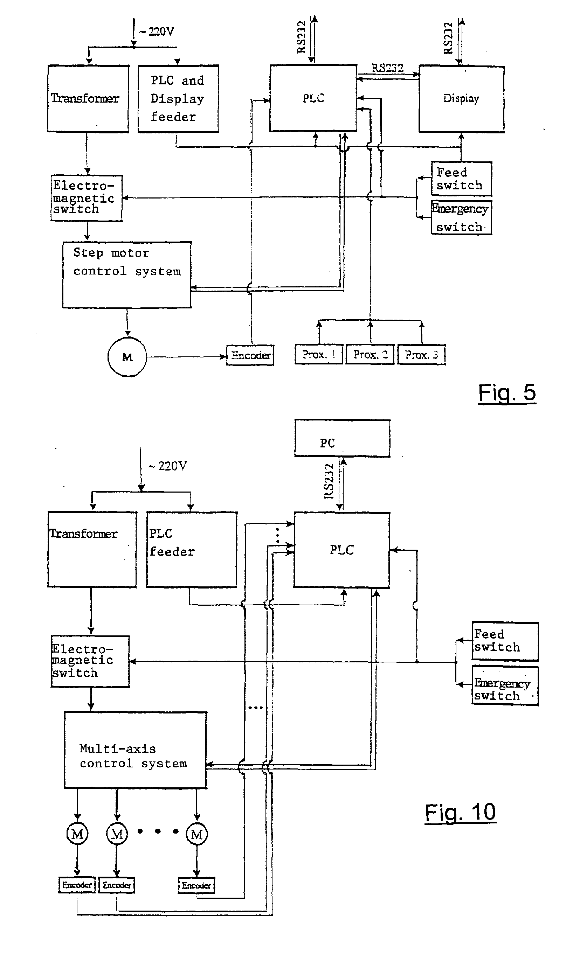

The electromechanical layout of the device is sketched in the flowchart diagram of figure 5, not described in detail since it can be immediately and obviously understood by a skilled person. In the diagram, sensors 3 and the relevant connections to the PLC are omitted.

Nonetheless, it will be noticed that the whole system is fed via a conventional connection with a 220V electric network and that the step motor is operated by means of a 64V transformer associated to the motor via an electromagnetic switch. The operation control unit is fed through a second transformer, not shown. The feed and stop switches respectively set the electromagnetic switch on and off, connecting and disconnecting the power supply to the step motor.

The connection between the PLC and the keyboard/display user interface - for entering the control inputs of the measurement operations - is carried out by means of a standard RS232 serial connection. The system further comprises two additional RS232 serial ports, one on the PLC and the other on the interface, for programming the PC.

A second and more advanced embodiment of the invention provides for the making of an electronically controlled multi-axis mechanical device, capable of simulating a practically limitless number of leg shapes, corresponding to respective anatomic attitudes, and of establishing a map of the compression exerted - even substantially point by point - by the pantyhose or the like when said attitudes are taken on by the wearer.

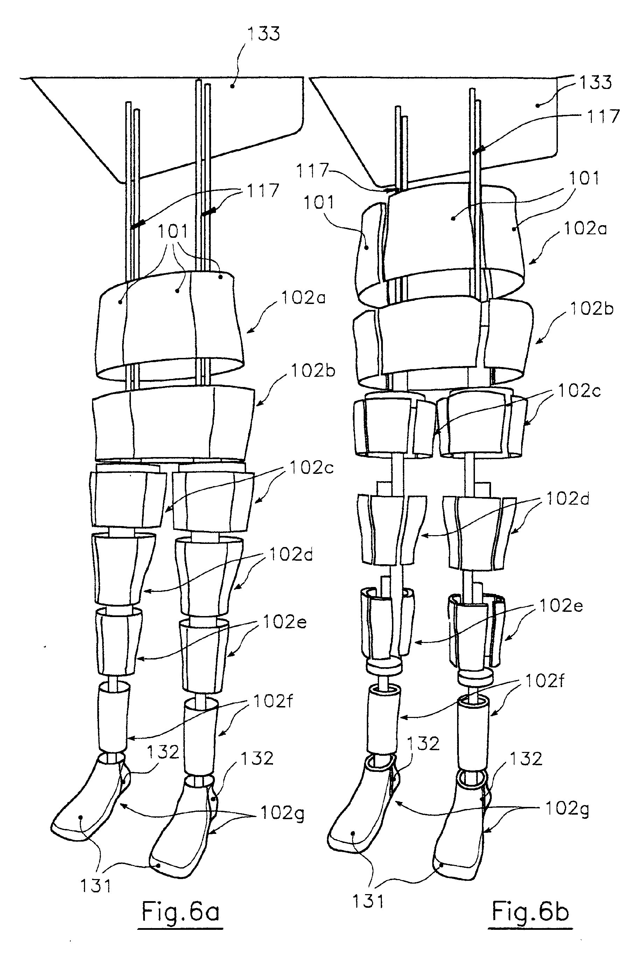

The overall shape of such device is shown in figures 6a and 6b, respectively when a minimum and a maximum size of the wearer are simulated. It can be easily appreciated that the lower part of a human body - i. e. that on which a pantyhose is worn - is reproduced thanks to an assembly of a number of members 102 made of movable shells, provided with sensors (not represented), suitable for carrying out a measurement of the compression according to the above described approach.

More precisely, the following members can be distinguished successively from top to bottom: members 102a and 102b, each formed by four shells 101 according to an arrangement which is completely equivalent to that of drum 2 of the first embodiment, reproducing the anatomic part corresponding to the body portion of a pantyhose; pairs of members 102c, 102d and 102e, operationally analogous to the above mentioned drum 2 either, and a pair of radially unextensible tubular members 102f, reproducing on the whole the legs of a wearer; and a pair of end members 102g, each formed by two shells 131, 132 with respective sensors, reproducing the feet of the wearer, as will be made clearer hereinafter.

Members from 102a to 102e are slidably supported by a frame comprising a couple of braces 117, extending downward from a support block 133, which the electronic control unit of the device, not shown, is associated to. In this way, the simulation of the various shapes can exploit also the possibility of varying the mutual displacement of said members in a longitudinal direction. The simulation is made more sophisticated also thanks to the fact that the various shells are shaped so as to comply with the CETME standards, known to the operators of the field. Then, the device can perfectly simulate standard anatomic contours varying from size I to VII, as identified by the so-called "British Standard" system, well known to the operators of the field either.

The radial displacement of the shells and the longitudinal one of the entire members are operated by first and second operating means, respectively. In further detail, as for the means actually provided for in the example, means 102a and 102b are operated - e.g. for reproducing broader or narrower hips or particular bottom shapes - through radial sliding guides and miniaturised step motors, having obvious features for a skilled person and thus neither shown nor specifically described.

Conversely, as far as members 102c, 102d and 102e are concerned, the limited space available demands the adoption of particular solutions, described hereinafter.

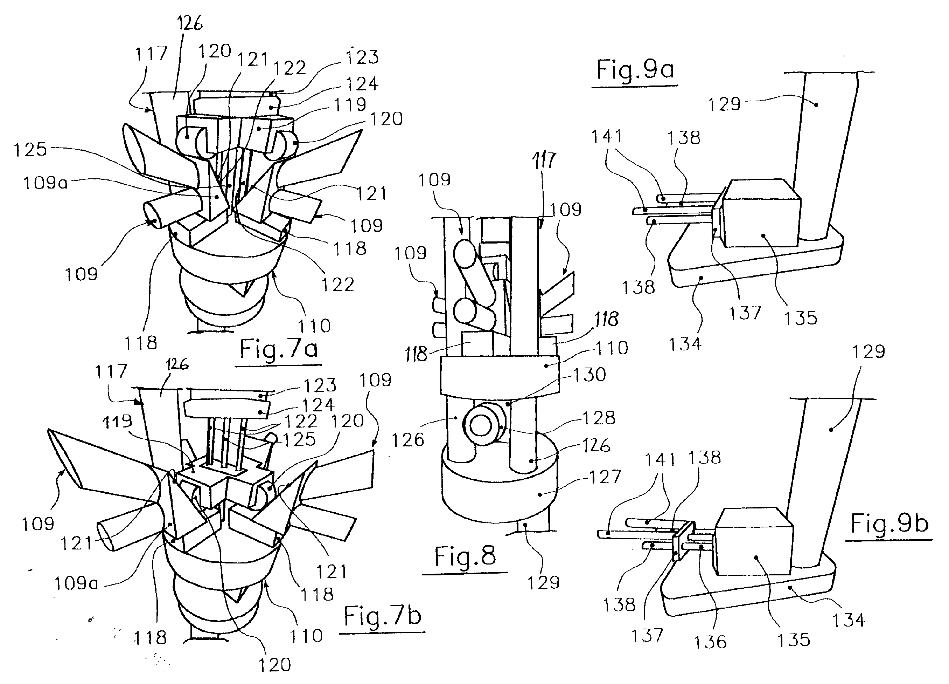

With reference to figures 7a, 7b and 8 one of the members just cited, namely member 102 substantially corresponding to the calf, is represented with its four shells lacking, for the sake of clarity. However, supports 109 to which the shells are connected can be seen. Such supports are slidable along ball-bearing radial guides 118, extending on the top face of a platform 110, slidably associated to one of the vertical braces 117. Supports 109 provide respective wedge-shaped inner portions 109a, defining faces 121 inclined with respect to the central longitudinal axis of the member, departing therefrom when the bottom-top direction is assumed for reference.

A slider 119 is supported by platform 110, via a couple of guides 122 extending upwards. Slider 119 provides four rollers 120, of which the axes are orthogonal two by two, each abutting against a respective inclined face 121 and co-operating therewith in order to cause the radial outwards displacement of supports 109, when slider 119 is lowered towards platform 110.

The latter operation is accomplished by a miniaturised step motor 123, connected to a fixed plate 214 integral to the upper end of vertical guides 122, and driving an endless screw 125 engaging with slider 119.

In the arrangement shown in figure 7a slider 119 is in an upper run-end position and supports 109 are held by springs - not shown - in a position which corresponds to the minimum overall width of the member, and thus to the minimum size that can be simulated. When motor 123 triggers the downwards displacement of slider 119, rollers 120 abut on inclined faces 121, causing supports 109 to slide, and consequently the entire member to extend radially until he maximum size width is reached (figure 7b). In this respect, it will be appreciated that different expansion paces and global amounts can be obtained by suitably varying the slant of inclined faces 121.

With reference now also to figure 8, member 102e is seen from a different angle with respect to figures 7a and 7b, in order to show the mechanism which permits the vertical operation of platform 110 and thus of the whole member. In such figure it can be noticed that a vertical brace 117 comprise a couple of guides 126, only one of which is represented in the previous figure for the sake of clarity, due to structural symmetry reasons. Both guides 126, along which platform 110 slides, end at the lower side in a further fixed platform 127. A post 129, actually a continuation of one of guides 126, extend from platform 127 and supports tubular member 102f and foot member 102g. One of the guides 126 is provided with a rack, not shown, engaging with a pinion 128 driven by a miniaturised step motor 130 integral to the lower face of platform 110.

Foot member 102g, shown in figures 9a and 9b, is a remarkable distinctive feature of the device according to this embodiment, primarily in light of the fact that, as mentioned in the introductory part, the traditional tool for measuring the compression, besides being unable to provide information about the compression exerted by the tip of the tubular portion, can not emulate any change in shape of the foot, whereby when a size I is simulated, the foot has the same shape as in a size VII simulation.

The above remarkably affects the references as far as the other parts of the tubular portion are concerned, thereby for instance the part which should correspond to the calf is displaced from its appropriate position and consequently the measurement of the compression thereof is carried out referring to a wrong circumference.

In foot member 102g a simple pair of shells is provided for accomplishing a single longitudinal extension movement. This because the foot width variation among the different sizes is substantially negligible, and such that the structural complexity involved by an arrangement similar to that of the other members would not be justified.

In greater detail, body 102g comprises a base 134, integral to post 129 supporting a step motor 135, making an element 137 slide along two horizontal guides 138, by means of an endless screw 136. A movable front shell 131 and a fixed back shell 132, not represented in figures 9a and 9b for the sake of clarity but visible in figures 6a and 6b, are supported by arms 141 extending horizontally from member 137, and by base 134, respectively.

Tubular member 129f, corresponding to the ankle area, is visible only in figures 6a and 6b and is integrally connected to post 129. It is unextensible, because the anatomical variations among different sizes are negligible in that area.

The electromechanical layout of the device according to the second embodiment of the invention is sketched in the flowchart diagram of figure 10, which in this case also, besides omitting the sensors network, is not described in detail since it can be immediately and obviously understood by a skilled person. However, it should be noted that such layout is analogous to that of the first embodiment, the difference being that in this case a multi-axis system for controlling and operating the various miniaturised step motors is used. The encoders, possibly embodied in the respective motors, are necessary in this embodiment, avoiding the use of a proximity sensor for each movement. Moreover, the user interface of the system is a PC - connected with the PLC via a serial port - which is entitled to the control of the device through a suitable software.

Such software, for controlling the model working procedures described hereinafter, is designed according to straightforward notions which, *per se,* do not form part of the invention and are not explained in detail. Said procedures, even though specifically referred to the second embodiment of the invention, can be carried out also in the device according to the simpler embodiment, with suitable changes and on the basis of elementary analogic considerations.

The first step to be accomplished in order to assure a correct operation of the system relates to the adjustment of the sensors. Namely, it is necessary to check whether the output of each sensor does not vary when the same force is applied thereon. The sensors are balanced directly on the shells that they are attached to. The balancing and adjustment operations are accomplished once and for all, after the sensors have been applied to the respective shells, by means of a suitably shaped instrument of a known type for analogous applications. Such tool can be flexible, e.g. provided with air chambers like those used for measuring the blood pressure, so as to be able to fit perfectly the irregular shapes of the shells.

Then, the output curve of each sensor is assumed to be time-insensitive. Such an assumption is certainly a reasonable one, considering that the output of a sensor is a function of its geometry and of its chemical/physical characteristics, parameters that are negligibly affected by the passing of time. The only change that can theoretically occur is a slight displacement of the null point of the sensor. In order to counterweigh this effect, which in any case affects only the absolute results and not the comparative ones between different articles, regular checks can be performed, by using the same adjustment instrument, so that a correction factor can be possibly recorded in the control software.

Before the measurement starts, the device accomplishes a self-learning cycle in order to establish automatically the maximum runs that the various mechanisms can perform. At the end of the cycle the device becomes completely closed, in an arrangement corresponding to a minimum size, so as to permit the manual loading of a pantyhose or another hosiery article by an operator.

The device is now ready for performing an extension cycle, suitably programmed as a function of the article to be measured, with the aim of removing the possible internal stresses caused by the manual loading of the pantyhose.

Then, the measurement operations follow programs that can be chosen by the user. After entering an identifying code of the product under analysis, a menu is made available, permitting to select the operation to be accomplished. The characterising data of each operation (number of cycles, run and speed) can be varied by the user and saved on a standard data carrier. In this way, the same experimental conditions can be reproduced for possible future comparisons. Furthermore, a whole sequence - each with its own data - can be saved so as to simulate a number of actual actions which one can perform, e.g. stand up and sit down, walk, ride a bicycle etc..

Obviously, it is the operator's task to create beforehand his own data base of measurement sequences, with the data that are suitable for simulating such actions. Hereinafter, some possible measurement programs are described.

### Static measurements

These are very quick measurements consisting in the assessment of the compression exerted by the pantyhose when it is extended to a predetermined width. In other words, simply the compression of the tubular portion as a function of its circumference is detected. Usually, the width corresponds to the size for which the article has been designed. In the PLC internal memory, in buffer areas, the data corresponding to the various sizes (standard or personalised ones) are stored. The members are automatically placed at the established co-ordinates and the compression can be directly detected. When static measurements are carried out, it is possible to change the width via step by step motions or to get to the predetermined width directly.

### Dynamic measurements

The dynamic measurements are extremely important, for the following reasons. It often occurs that the anatomy of a given body differs from the standard corresponding to a determined size. It is desirable to analyse the performance of a pantyhose in non-standard conditions too, i. e. when a pantyhose of a certain size is extended to a width differing from the estimated value. The analysis of the non-standard performance is particularly important when one-sized knee-highs are considered, in that case being fundamental to assess the elastic response as a function of the width of the area under consideration.

The system also permits to scan the compression of the pantyhose or the like when the width varies between two values. In case of knee-highs, a very wide scan is set, so as to cover all the possible anatomies. If it is desired to evaluate the performance around a determined size, a suitable scan is set around the corresponding standard value.

A wider scan than in the previous case is set when it is required to evaluate the elastic response of an article designed for a certain size but worn by a wearer whose anatomy is different from the standard of said size.

In any case, the data of the scans can be set at will. Besides and advantageously, programs corresponding to the various desired measurements can be stored. All the measurements are recorded both in extension and shrinkage, for obtaining hysteresis curves that can be easily studied both through the internal software of the system, and with any other suitable software available on the market.

The hysteresis curves are the most appropriate tool for evaluating the elastic response of the product. A very important achievement of the scan-type measurement is the creation of a very extensive data base, bearing in mind that the system can manage a sampling frequency of 8Hz. When multiple cycles are performed between two boundary widths, it is possible to obtain the average of the various measurements so as to make the noise as low as possible.

### Strength dynamic measurements

A further advantage of the present device is that the elastic response of the pantyhose or the like can be evaluated as time passes, simulating the repeated use of the article. By setting repeated shrinkage cycles it is possible to record the performance of the pantyhose in time, focusing on the maximum and minimum values of the compression, or storing completely all the groups of data.

### Simulations of the leg motion

After studying the elastic response of the product as a function of the extension to which it is subjected, it is possible to simulate the stress during the leg motion by setting suitably wide scans around a certain width.

It is clear from the above that the device according to the present invention, in particular in the second embodiment, allows the accomplishment of very important objects in the hosiery field, overcoming the prior art background with a means which is very useful both when new articles are studied and designed, and when the same articles are statistically checked in the manufacturing phase.

Extremely accurate measurements can be carried out, and, above all, absolute ones, that is to say not limited to comparisons between two or more products. In other words, the device can provide - even point by point - the compression that is actually exerted when a pantyhose, or the like, is worn.

Variations and/or modifications can be brought to device for measuring the compression that can be exerted by a hosiery article according to the present invention without departing from the scope of the invention itself as defined in the appended claims. Namely, the radial extensibility of drum 2 and of members 102 can be accomplished with technical solutions which are different from those described, but functionally equivalent thereto.

## Claims

1. A device for measuring the compression that can be exerted by a hosiery article, characterised in that it comprises at least one axially symmetrical and radially extensible member (2, 102), on the outside of which said article is to be arranged, two-dimensional sensors (3), each comprising a matrix arrangement of load cells, connected to the outside of said member (2, 102), first operating means mechanically connected to said member (2, 102) for the radial extension thereof, and programmable control means, for controlling said first operating means according to predetermined working steps, and for reading and processing the output signals of said sensors (3).

2. The device according to claim 1, wherein said member (2, 102) consists of a number of rigid shells (1, 101) each supporting a respective sensor (3).

3. The device according to claim 2, wherein said shells (1) form a single, substantially cylindrical member (2) and are supported by jaws (9) of a self-centering arrangement (10), mechanically connected to said first operating means.

4. The device according to claim (2), wherein said first operating means comprise a step motor.

5. The device according to claim 1 or 2, comprising a number of extensible members (102), on the whole reproducing at least one leg of a wearer, each member (102) being associated to respective first operating means which can be controlled independently from each other.

6. The device according to claim 5, comprising a frame, at least an upper extensible member (102a, 102b) slidably supported by said frame, for reproducing the anatomic part corresponding to the body portion of a pantyhose, a number of members in pairs (102c, 102d, 102e), slidably supported by said frame, each member of the pair reproducing a respective longitudinal portion of the leg, and a couple of members (102g) fixed to said frame, for reproducing the feet of the wearer, second operating means being associated to the slidable members (102a, 102b, 102c, 102d, 102e), for controlling the axial displacement thereof along said frame, independently from each other.

7. The device according to claim 6, wherein each slidable leg-portion member (102c, 102d, 102e) comprises a platform (110) slidably supported by said frame, supports (109) for holding said shells, radially slidable on said platform (110), elastic means co-operating with said supports (109) for hindering the displacement thereof from the centre of the platform (110), and a motor-driven slider (119), coaxially and slidably supported by said platform (110), abutting against inclined faces (121) of said supports (109) in order to overcome the effect of said elastic means.

8. The device according to claim 7, wherein said slider (119) has a number of rollers (120) for abutment against respective inclined faces (121) of said support (109).

9. The device according to claim 7 or 8, wherein said second operating means comprise pinion (128) and rack means associated respectively to said platform (110) and said frame.

10. The device according to any of the claims from 7 to 9, wherein each of said foot members (102g) comprises a fixed back shell (132), connected to a lower end of said frame, and a movable front shell (131), slidable frontward with respect to said back shell (132).
